# FASCICULE DE BREVET EUROPEEN

(11) **EP 0 711 778 B1**
(45) Date de publication et mention de la délivrance du brevet: **10.12.1997**
(21) Numéro de dépôt: 95402227.3
(22) Date de dépôt: 05.10.1995
(51) Int. Cl.: C07F 7/08

(54) **Nouveaux filtres solaires, compositions photoprotectrices les contenant et utilisations**
Sonnenfilter, diese enthaltende Sonnenschutzzusammensetzungen und deren Verwendung
Sunscreens, sunscreen compositions and use thereof

(30) Priorité: 08.11.1994 FR 9413394
(43) Date de publication de la demande: 15.05.1996
(73) Titulaire: L'OREAL, 75008 Paris (FR)
(72) Inventeur: Richard, Hervé, F-93420 Villepinte (FR); Leduc, Madeleine, Résidence "Les Chèvrefeuilles", F-75011 Paris (FR); Lagrange, Alain, F-77700 Couvray (FR)
(74) Mandataire: Andral, Christophe André Louis

(56) Documents cités:
- EP-A- 0 282 294
- EP-A- 0 392 883
- CHEMICAL ABSTRACTS, vol. 113, no. 16, 15 Octobre 1990 Columbus, Ohio, US; abstract no. 133518y, NAKAHARA, Y. ET AL. 'LIGHT-RESISTANT POLYMER COMPOSITION' page 52;

## Description

La présente invention concerne de nouveaux dérivés triorganosilaniques de benzotriazoles plus particulièrement utilisables à titre de filtres organiques solaires dans des compositions cosmétiques destinées à la protection de la peau et des cheveux contre le rayonnement ultraviolet. L'invention concerne également l'utilisation desdits composés dans l'application cosmétique susmentionnée, ainsi que les compositions cosmétiques à propriétés améliorées les contenant.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreux composés destinées à la photoprotection (UV-A et/ou UV-B) de la peau ont été proposées à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber selectivement les rayonnements UV nocifs, ces filtres (et leurs quantités) étant sélectionnés en fonction de l'indice de protection recherché (l'indice de protection (lP) s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à l'eau et à la transpiration (rémanence).

Parmi tous les composés aromatiques qui ont été préconisés à cet effet, on peut citer notamment les dérivés de l'acide p- aminobenzoïque, les dérivés du benzylidènecamphre, les dérivés de l'acide cinnamique et les dérivés du benzotriazole. Cependant, certaines de ces substances ne présentent pas toutes les propriétés requises pour une utilisation convenable comme filtres UV dans les compositions antisolaires. En particulier, leur pouvoir filtrant intrinsèque peut être insuffisant, leur solubilité dans les différents type de formulations utilisés en matière de protection solaire n'est pas toujours suffisamment bonne (liposolubilité notamment), elles peuvent ne pas posséder une stabilité suffisante à la lumière (photostabilité) et elles peuvent également présenter une mauvaise résistance à l'eau et à la sueur. Il est également souhaitable que ces substances filtrantes ne pénètrent pas dans la peau.

Ainsi, dans le cas plus particulier des substances filtres de type benzotriazole, on a cherché à obtenir des produits à propriétés améliorées, en particulier au niveau de leur liposolubilité et de leur caractère cosmétique, en venant fixer par greffage (hydrosilylation) le groupement filtrant benzotriazole sur une chaine macromoléculaire de type silicone (organopolysiloxane). Cette technique, décrite dans la demande de brevet EP 0 392 883 au nom de la Demanderesse, conduit certes à des composés intéressants (ces produits sont connus sous le nom général de "silicones filtres"), mais le caractère liposoluble de ces derniers peut encore apparaitre comme insuffisant et, de plus, afin d'obtenir des propriétés filtrantes convenables avec ce type de produits, il est souvent nécessaire de mettre en oeuvre des quantités relativement importantes de ces polymères filtres, ce qui se traduit par de mauvaises propriétés cosmétiques au niveau des formulations qui les contiennent. Enfin, la stabilité, et en particulier la photostabilité, de tels produits n'est pas encore tout à fait satisfaisante.

La présente invention vise à résoudre les problèmes ci-dessus en proposant de nouveaux composés, de type triorganosilanes, à motif benzotriazole particulier, qui présentent des propriétés améliorées, notamment au niveau de leur solubilité dans les corps gras, de leurs propriétés cosmétiques, et de leur stabilité, notamment chimique et photochimique.

Plus précisemment encore, il a été trouvé, selon la présente invention, qu'en associant, notamment par réaction d'hydrosylilation, un dérivé de benzotriazole particulier, à savoir plus précisément un benzotriazoles présentant une chaîne courte hydrocarbonée à insaturation éthylénique, avec un silane particulier et convenablement sélectionné, il était possible d'aboutir à de nouveaux composés du type triorganosilanes filtres obviant aux inconvénients des silicones filtres de l'art antérieur, ces nouveaux composés présentant en particulier de très bonnes propriétés filtrantes, tant dans l'UV-A que dans l'UV-B, une très bonne solubilité dans les solvants organiques usuels et notamment les corps gras tels que les huiles, d'excellentes propriétés cosmétiques, une très bonne stabilité chimique (meilleure résistance à l'hydrolyse tant en milieu acide que basique) et photochimique, les rendant particulièrement appropriés à une utilisation comme filtres solaires dans des, ou pour la préparation de, compositions cosmétiques destinées à la protection de la peau et/ou des cheveux contre le rayonnement ultraviolet.

La présente invention a ainsi pour premier objet de nouveaux composés, à savoir des dérivés silaniques de benzotriazoles, qui sont caractérisés par le fait qu'ils répondent à la formule (1) suivante : 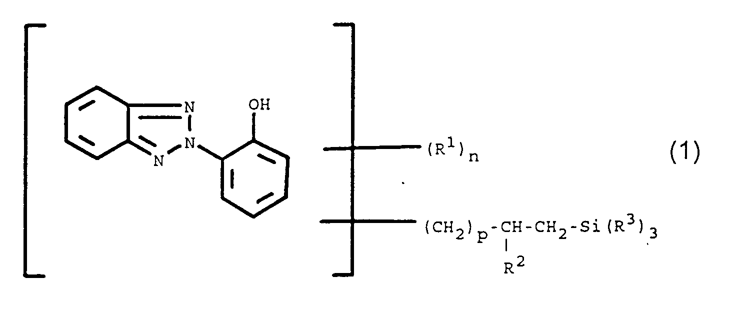 dans laquelle :
- n est un nombre entier compris entre 0 et 3 inclusivement et R¹, identiques ou différents, sont choisis parmi les radicaux alkyles en C₁-C₈,
- R² représente l'hydrogène ou un radical alkyle en C₁-C₄,
- R³, identiques ou différents, sont choisis parmi les radicaux alkyles en C₁-C₈, et phényle,
- p est un nombre entier compris entre 0 et 10 inclusivement.

Comme cela ressort de la formule (1) donnée ci-dessus, l'accrochage du chainon (CH₂)ₚ-CH(R²)-CH₂- sur le motif benzotriazole, qui assure donc le raccordement dudit motif benzotriazole à l'atome de silicium du triorganosilane, peut, selon la présente invention, se faire dans toutes les positions disponibles offertes par les deux noyaux aromatiques du benzotriazole :

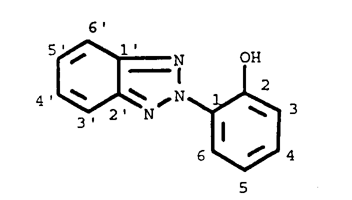

De préférence, cet accrochage se fait en position 3, 5 (noyau aromatique portant la fonction hydroxy) ou 4' (noyau benzénique adjacent le cycle triazolé), et encore plus préférentiellement en position 3 ou 5.

De même, l'accrochage du ou des motifs subsituants R¹, lorsque présent(s), peut se faire dans toutes les autres positions disponibles au sein du benzotriazole. Toutefois, de préférence, cet accrochage se fait en position 3, 4' et/ou 5.

Dans la formule (1) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle préférés selon l'invention sont les radicaux méthyle, éthyle, propyle, n-butyle, n-octyle et éthyl-2 hexyle. Encore plus préférentiellement, les radicaux R¹ et R² sont des radicaux méthyle.

Parmi les composés de formule (1) rentrant dans le cadre de la présente invention, on préfère plus particulièrement les dérivés statistiques ou bien définis à blocs présentant au moins l'une, et encore plus préférentiellement l'ensemble, des caractéristiques suivantes :
- R¹ est méthyle
- R² est un atome d'hydrogène ou est méthyle
- R³ est méthyle, éthyle, isopropyle ou isobutyle
- p est égal à 1.

Comme exemples de dérivés silaniques de benzotriazoles plus particulièrement visés par la présente invention, on peut notamment citer les :
a) 2-(2'-hydroxy-5'-méthyl-3'-triisobutylsilanyl propyl phényl)-2H-benzotriazole
b) 2-[2'-hydroxy-3'-(3-diéthylméthylsilanyl-2-méthyl propyl)-5'-méthyl phényl]- 2H-benzotriazole

Pour préparer les composés de formule (1), on peut procéder classiquement en mettant en oeuvre une réaction d'hydrosylilation du type 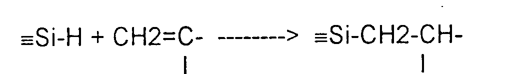 à partir du triorganosilane correspondant dans lequel le radical A est remplacé par un atome d'hydrogène. Ces dérivés organiques silaniques à fonction SiH sont des produits bien connus dans l'industrie des silanes et sont par ailleurs généralement disponibles dans le commerce.

Ces triorganosilanes répondent donc à la formule (2) suivante :

H-Si(R³)₃ (2)

dans laquelle R³ a la même signification que celle donnée pour la formule (1).

Sur ce dérivé silanique de formule (2), on effectue donc une réaction d'hydrosilylation classique, opérée en présence d'une quantité catalytiquement efficace d'un catalyseur au platine, sur un dérivé organique de benzotriazole de formule (3) suivante : 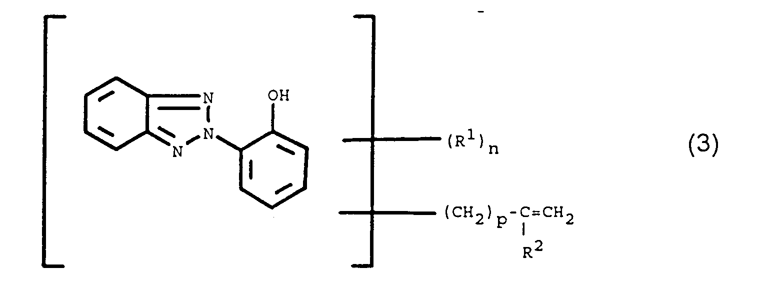 dans laquelle R¹, R², n et p ont la même signification que celle donnée ci-dessus pour la formule (1).

Des procédés convenant à la préparation des produits de formule (3) ci-dessus sont notamment décrits dans les brevets US- 4 316 033 et US- 4 328 346, ainsi que dans la demande EP-A- 0 392 883 précitée.

En outre, les détails des conditions opératoires à suivre pour conduire la réaction d'hydrosylilation entre les composés de formule (2) ci-dessus avec le composé de formule (3) ci-dessus sont donnés dans la demande de brevet EP- 0 392 883 précitée, dont l'enseignement est, à cet égard, totalement inclus à titre de référence dans la présente description.

Par rapport aux silicones filtres de l'art antérieur telles que décrites dans la demande de brevet EP 0 392 883, les filtres selon l'invention présentent donc au moins une différence structurelle essentielle à l'origine de leurs propriétés remarquables, à savoir celle reposant sur le fait que, d'une part, il n'existe qu'un seul motif benzotriazole greffé sur une chaine siliconée et que, d'autre part, cette chaine siliconée est ici réduite à sa plus simple expression, puisqu'il s'agit, selon l'invention, d'un monosilane.

Comme indiqué précédemment, les composés de formule (1) ci-dessus présentent un excellent pouvoir filtrant intrinsèque à l'égard du rayonnement ultraviolet UV-A et UV-B. En outre, de par leur caractère fortement liposoluble, les composés de formule (1) peuvent être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace. De plus, leurs propriétés cosmétiques sont très bonnes : en particulier, par rapport aux silicones filtres de l'art antérieur, ces produits sont moins collants et apportent plus de douceur. Enfin, ces produits sont très stables chimiquement (ils résistent notamment particulièrement bien aux dégradations par hydrolyse susceptibles d'intervenir tant en milieux acides que basiques), et photochimiquement (c'est à dire qu'ils résistent en outre bien aux dégradations induites par des rayons ultraviolets).

La présente invention a donc aussi pour objet une composition cosmétique comprenant, dans un support cosmétiquement acceptable contenant de préférence au moins une phase grasse ou un solvant organique, une quantité efficace d'au moins un composé de formules (1) définie ci-avant.
Les composés de formules (1) sont généralement présents dans des proportions comprises entre 0,1 % et 20 % en poids, de préférence entre 0,5 % et 10 % en poids, par rapport au poids total de la composition.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et eventuellement être conditionnée en aérosol et se présenter sous forme de mousse ou de spray.

Elle peut contenir les adjuvants cosmétiques habituellement utilisés dans le domaine, tels que des corps gras, des solvants organiques, des silicones, des épaississants, des adoucissants, des filtres solaires complémentaires, des agents anti-mousses, des agents hydratants, des parfums, des conservateurs, des tensio-actifs, des charges, des sequestrants, des polymères anioniques, cationiques, non ioniques ou amphotères ou leurs mélanges, des propulseurs, des agents alcalinisants ou acidifiants, des colorants, des pigments ou nanopigments en particulier ceux destinés à assurer un effet photoprotecteur complémentaire par blocage physique du rayonnement ultraviolet, ou tout autre ingrédient habituellement utilisé en cosmétique, en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol.

Les corps gras peuvent être constitués par une huile ou par une cire ou leurs mélanges, des acides gras, des esters d'acides gras, des alcools gras, la vaseline, la paraffine, la lanoline, la lanolinehydrogénée, la lanoline acétylée. Les huiles peuvent être choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicones, volatiles ou non, et les isoparaffines.

Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras, ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est utilisée comme produit de maquillage des cils, des sourcils, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a aussi pour objet un procédé de protection de la peau et cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (1) tel que défini ci-avant.

Les exemples qui suivent illustrent l'invention sans toutefois en limiter la portée.

### EXEMPLE 1

Cet exemple illustre la préparation d'un composé conforme à l'invention de formule : 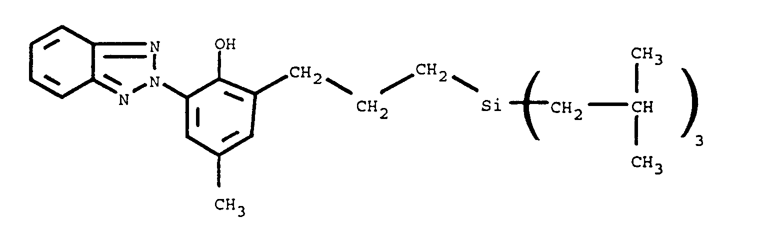 (ce produit correspond à un composé de formule (1) pour lequel : n = 1 et R¹ est méthyle ; p = 1 ; R² = H ; R³ = isobutyle).
Dans un ballon tout équipé, on charge 20 g (0,075 mole) de 2-allyl-6-benzotriazol-2-yl-4-méthyl-phénol et 25 ml de toluène sec. Le mélange est porté à 90°C sous azote. On ajoute le catalyseur d'hydrosylilation (complexe à 3-3,5 % de Pt dans du cyclovinylméthylsiloxane de Hüls Petrarch PC085 : 300 µl) puis 15,1 g de tri-isobutylsilane en 1 heure. Après 24 heures à ébullition sous azote, on concentre le milieu réactionnel. Le dérivé de benzotriazole de départ n'ayant pas réagi est filtré et le silane de départ n'ayant pas réagi est distillé sous vide (0,2 mmHg). Le résidu obtenu est ensuite chromatographié sur silice (éluant : heptane). On récupère alors une fraction du produit final désiré, qui se présente sous la forme d'une poudre blanche de point de fusion 80-82°C.

Les caractéristiques en absorption UV (mesurées dans l'éthanol) de ce produit sont les suivantes :

| | |
|---|---|
| λₘₐₓ : 305 nm | εₘₐₓ : 15 400 |
| λₘₐₓ : 342 nm | εₘₐₓ : 14 500 |

Ce produit peut donc être utilisé très efficacement à titre de filtre solaire actif dans l'UV-A et l'UV-B.

### EXEMPLE 2

Cet exemple illustre la préparation d'un autre composé conforme à l'invention de formule : 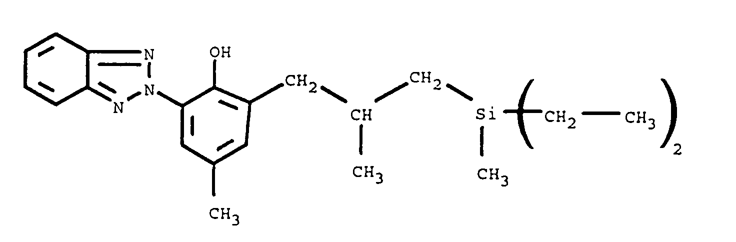 (ce produit correspond à un composé de formule (1) pour lequel : n = 1 et R¹ est méthyle ; p = 1 ; R² = méthyle ; un R³ = méthyle et deux R³ = éthyle).
Dans un ballon tout équipé, on charge 13,97 g (0,05 mole) de 2-benzotriazol-2-yl-4-méthyl-6-(2-méthyl-allyl)-phénol et 25 ml de toluène sec. Le mélange est porté à 70°C sous azote. On ajoute le catalyseur d'hydrosylilation (complexe à 3-3,5 % de Pt dans du cyclovinylméthylsiloxane de Hüls Petrarch PC085 : 200 µl) puis 5,37 g (0,0525 mole) de diéthyl méthyl silane. Après 10 heures à 80°C sous azote, on concentre le milieu réactionnel et on effectue une chromatographie sur silice sous pression (éluant : heptane). On récupère alors une fraction du produit final désiré, qui se présente sous la forme d'une poudre blanche de point de fusion 37°C.

Les caractéristiques en absorption UV (mesurées dans l'éthanol) de ce produit sont les suivantes :

| | |
|---|---|
| λₘₐₓ : 304 nm | εₘₐₓ : 15 520 |
| λₘₐₓ : 343 nm | εₘₐₓ : 15 050 |

Ce produit peut donc être utilisé très efficacement à titre de filtre solaire actif dans l'UV-A et l'UV-B.

### EXEMPLE 3

On illustre ici une formulation concrète de composition cosmétique antisolaire conforme à l'invention, à savoir une crème antisolaire.

| | |
|---|---|
| - Composé de l'exemple 1 | 5 g |
| - Mélange d'alcool cétylstéarylique et d'alcool cétylstéarylique oxyéthyléné à 33 moles d'OE ("SINNOVAX AO" de chez HENKEL) | 7 g |
| - Mélange de mono et distéarate de glycérol non autoémulsifiable | 2 g |
| - Alcool cétylique | 1,5 g |
| - Benzoate d'alcools en C₁₂-C₁₅ ("FINSOLV TN" de chez WITCO) | 20 g |
| - Polydiméthylsiloxane | 1,5 g |
| - Glycérine | 17,5 g |
| - Parfum, conservateur qs | |
| - Eau qsp | 100 g |

Cette crème est préparée selon les techniques classiques de préparation d'émulsions en dissolvant le filtre dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40 °C on ajoute enfin parfum et conservateur.

## Revendications

1. composés de formule : 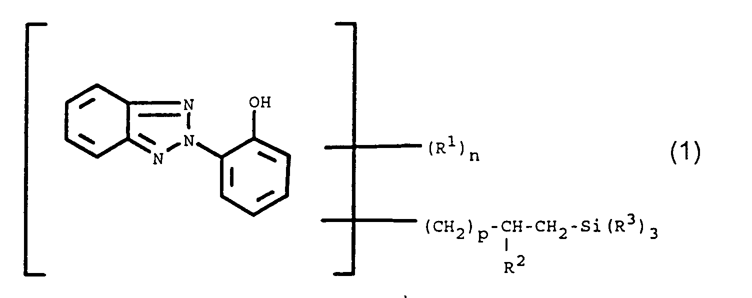 dans laquelle :
- n est un nombre entier compris entre 0 et 3 inclusivement et R¹, identiques ou différents, sont choisis parmi les radicaux alkyles en C₁-C₈,
- R² représente l'hydrogène ou un radical alkyle en C₁-C₄,
- R³, identiques ou différents, sont choisis parmi les radicaux alkyles en C₁-C₈, et phényle,
- p est un nombre entier compris entre 0 et 10 inclusivement.

2. composés selon la revendication 1, caractérisés par le fait que les radicaux alkyles sont choisis parmi les radicaux méthyle, éthyle, propyle, n-butyle, n-octyle ou éthyl-2 hexyle.

3. composés selon l'une quelconque des revendications 1 ou 2, caractérisés par le fait que R¹ est méthyle.

4. composés selon l'une quelconque des revendications précédentes, caractérisés par le fait que R² est méthyle ou hydrogène.

5. composés selon l'une quelconque des revendications précédentes, caractérisés par le fait que R³ est méthyle, éthyle, isopropyle ou isobutyle.

6. composés selon l'une quelconque des revendications précédentes, caractérisés par le fait que p est égal à 1.

7. composés selon l'une quelconque des revendications précédentes, caractérisés par le fait que l'accrochage du chainon -(CH₂)ₚ-CH(R²)-CH₂- sur le motif benzotriazole se fait en position 3, 4' ou 5 de ce dernier.

8. composés selon la revendication 7, caractérisés par le fait que ledit accrochage se fait en position 3 ou 5.

9. composés selon l'une quelconque des revendications précédentes, caractérisés par le fait que l'accrochage du substituant R¹ sur le motif benzotriazole se fait en position(s) 3, 4' et/ou 5 de ce dernier.

10. Utilisation des composés de formule (1) ) tels que définis à l'une quelconque des revendications 1 à 9, à titre de filtres solaires actifs dans le domaine des UV-A et UV-B.

11. Composition cosmétique, en particulier pour la filtration des rayons ultraviolets, caractérisée par le fait qu'elle comprend, dans un support cosmétiquement acceptable, une quantité efficace d'au moins un des composés définis à l'une quelconque des revendications 1 à 9.

12. Composition cosmétique selon la revendication 11, caractérisée par le fait que ledit support cosmétiquement acceptable contient au moins une phase grasse ou un solvant organique.

13. Composition cosmétique selon la revendication 12, caractérisée par le fait que ledit support se présente sous la forme d'une émulsion de type huile-dans-eau ou eau-dans-huile, de préférence huile-dans-eau.

14. Composition cosmétique selon l'une quelconque des revendications 11 à 13, caractérisée par le fait que la teneur en composé(s) filtrant(s) est comprise entre 0,1 et 20 % en poids par rapport au poids total de la composition.

15. Composition cosmétique selon la revendication 14, caractérisée par le fait que ladite teneur est comprise entre entre 0,5 et 10 % en poids.

16. Procédé de protection de la peau et/ou des cheveux contre le rayonnement ultraviolet, en particulier le rayonnement solaire, caractérisé par le fait qu'il consiste à appliquer sur la peau et/ou les cheveux une quantité efficace d'au moins un composé ou d'au moins une composition tels que définis à l'une quelconque des revendications précédentes.

## Claims

1. Compounds of formula: 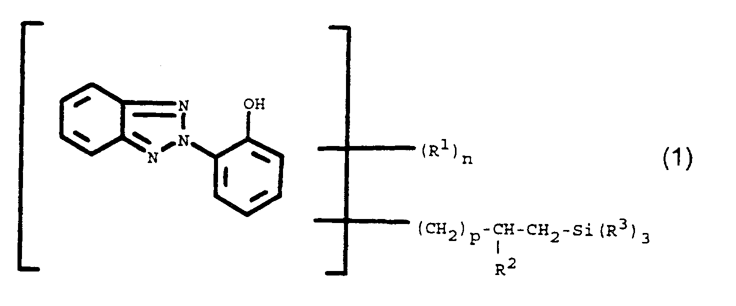 in which
- n is an integer between 0 and 3 inclusively and the groups R¹, which may be identical or different, are chosen from C₁-C₈ alkyl radicals,
- R² represents hydrogen or a C₁-C₄ alkyl radical,
- the groups R³, which may be identical or different, are chosen from C₁-C₈ alkyl radicals, and phenyl radicals,
- p is an integer between 0 and 10 inclusively.

2. Compounds according to Claim 1, characterized in that the alkyl radicals are chosen from methyl, ethyl, propyl, n-butyl, n-octyl and 2-ethylhexyl radicals.

3. Compounds according to either of Claims 1 and 2, characterized in that R¹ is methyl.

4. Compounds according to any one of the preceding claims, characterized in that R² is methyl or hydrogen.

5. Compounds according to any one of the preceding claims, characterized in that R³ is methyl, ethyl, isopropyl or isobutyl.

6. Compounds according to any one of the preceding claims, characterized in that p is equal to 1.

7. Compounds according to any one of the preceding claims, characterized in that the chain unit -(CH₂)ₚ-CH(R²)-CH₂- is anchored onto the benzotriazole unit in position 3, 4' or 5 of the latter.

8. Compounds according to Claim 7, characterized in that the said anchoring takes place in position 3 or 5.

9. Compounds according to any one of the preceding claims, characterized in that the substituent R¹ is anchored onto the benzotriazole unit in position(s) 3, 4' and/or 5 of the latter.

10. Use of the compounds of formula (1) as defined in any one of Claims 1 to 9, as sunscreens which are active in the UV-A and UV-B region.

11. Cosmetic composition, in particular for the filtration of ultraviolet rays, characterized in that it comprises, in a cosmetically acceptable vehicle, an effective amount of at least one of the compounds defined in any one of Claims 1 to 9.

12. Cosmetic composition according to Claim 11, characterized in that the said cosmetically acceptable vehicle contains at least one fatty phase or one organic solvent.

13. Cosmetic composition according to Claim 12, characterized in that the said vehicle is in the form of an emulsion of oil-in-water or water-in-oil type, preferably of oil-in-water type.

14. Cosmetic composition according to any one of Claims 11 to 13, characterized in that the content of screening compound(s) is between 0.1 and 20 % by weight relative to the total weight of the composition.

15. Cosmetic composition according to Claim 14, characterized in that the said content is between 0.5 and 10 % by weight.

16. Process for the protection of the skin and/or the hair against ultraviolet radiation, in particular solar radiation, characterized in that it consists in applying to the skin and/or the hair an effective amount of at least one compound or of at least one composition as are defined in any one of the preceding claims.

## Patentansprüche

1. Verbindungen der Formel: 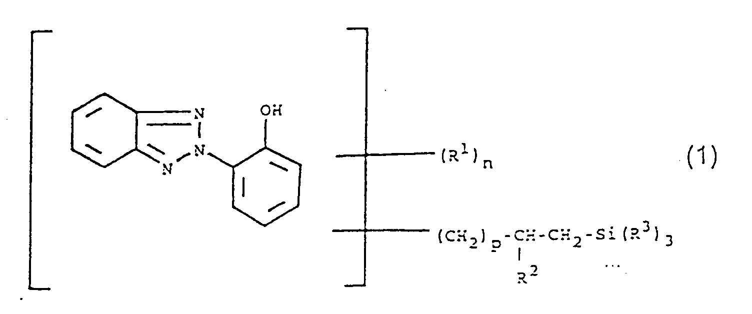 worin:
- n Null oder eine ganze Zahl im Bereich von 1 bis schließlich 3 bedeutet,
- die Gruppen R¹, die identisch oder voneinander verschieden sind, unter den C₁₋₈-Alkylgruppen ausgewählt sind
- R² Wasserstoff oder C₁₋₄-Alkyl bedeutet,
- die Gruppen R³, die identisch oder voneinander verschieden sind, unter den C₁₋₈-Alkylgruppen und Phenyl ausgewählt sind, und
- p Null oder eine ganze Zahl im Bereich von 1 bis einschließlich 10 bedeutet.

2. Verbindungen nach Anspruch 1, dadurch gekennzeichnet, daß die Alkylgruppen unter den Gruppen Methyl, Ethyl, Propyl, n-Butyl, n-Octyl oder 2-Ethylhexyl ausgewählt sind.

3. Verbindungen nach einem der Ansprüche 1 oder 2, dadurch gekennzeichnet, daß die Gruppe R¹ Methyl ist.

4. Verbindungen nach einem der vorhergehenden Ansprüche, dadurch gekennzeichnet, daß R² Methyl oder Wasserstoff bedeutet.

5. Verbindungen nach einem der vorhergehenden Ansprüche, dadurch gekennzeichnet, daß R³ Methyl, Ethyl, Isopropyl oder Isobutyl bedeutet.

6. Verbindungen nach einem der vorhergehenden Ansprüche, dadurch gekennzeichnet, daß p 1 ist.

7. Verbindungen nach einem der vorhergehenden Ansprüche, dadurch gekennzeichnet, daß die Bindung der Kette -(CH₂)ₚ-CH(R²)-(CH₂)- an die Benzotriazoleinheit in den Stellungen 3, 4' oder 5 dieser Einheit erfolgt.

8. Verbindungen nach Anspruch 7, dadurch gekennzeichnet, daß die Bindung in der Stellung 3 oder 5 erfolgt.

9. Verbindungen nach einem der vorhergehenden Ansprüche, dadurch gekennzeichnet, daß die Bindung des Substituenten R¹ an der Benzotriazoleinheit in der Position oder den Positionen 3, 4' und/oder 5 der Benzotriazoleinheit erfolgt.

10. Verwendung der Verbindungen der Formel (1) nach einem der Ansprüche 1 bis 9 als im UV-A- und UV-B-Bereich wirksame Sonnenschutzfilter.

11. Kosmetische Zusammensetzungen, insbesondere Zusammensetzung zur Filtrierung der UV-Strahlung, dadurch gekennzeichnet, daß sie in einem kosmetisch akzeptablen Träger eine wirksame Menge mindestens einer Verbindung nach einem der Ansprüche 1 bis 9 enthält.

12. Kosmetische Zusammensetzung nach Anspruch 11, dadurch gekennzeichnet, daß der kosmetisch akzeptable Träger mindestens eine Fettphase oder ein organisches Losungsmittel enthält.

13. Kosmetische Zusammensetzung nach Anspruch 12, dadurch gekennzeichnet, daß der Träger in Form einer Öl-in-Wasser-Emulsion oder Wasser-in-Öl-Emulsion und vorzugsweise Öl-in-Wasser-Emulsion vorliegt.

14. Kosmetische Zusammensetzung nach einem der Ansprüche 11 bis 13, dadurch gekennzeichnet, daß der Gehalt an Filterverbindung oder Filterverbindungen im Bereich von 0,1 bis 20 Gew.-%, bezogen auf das Gesamtgewicht der Zusammensetzung, liegt.

15. Kosmetische Zusammensetzung nach Anspruch 14, dadurch gekennzeichnet, daß der Gehalt im Bereich von 0,5 bis 10 Gew.-% liegt.

16. Verfahren zum Schutz der Haut und/oder der Haare gegen UV-Strahlung, insbesondere gegen Sonnenlicht, dadurch gekennzeichnet, daß es darin besteht, auf die Haut und/oder die Haare eine wirksame Menge mindestens einer Verbindung oder mindestens einer Zusammensetzung nach einem der vorhergehenden Ansprüche aufzutragen.
